# EUROPEAN PATENT APPLICATION

(11) **EP 4 567 120 A1**
(43) Date of publication of application: **11.06.2025**
(21) Application number: 23215221.5
(22) Date of filing: 08.12.2023
(51) Int. Cl.: C12P 5/02, C12M 1/107, C12M 1/00, C12M 1/26, C12M 1/34

(54) **A METHOD AND A SYSTEM FOR PRODUCTION OF BIOGAS FROM LIGNOCELLULOSE MATERIAL**

(71) Applicant: Biofuel Technology Holding ApS, 8250 Egå (DK)
(72) Inventor: Bonde, Torben Andreas, 8250 Egå (DK); Hostrup, Martin, 2635 Ishøj (DK)
(74) Representative: Patrade A/S

(57) **Abstract**

The present invention relates to an integrally combined method and a corresponding plant for production of biogas from raw material comprising lignocellulose material, in particular from raw material with high amount of lignocellulose material in an energy and resource saving manner, by applying multiple fermentation steps and optimized recycling steps.

## Description

### Field of the Invention

The present invention relates to an integrally combined method and a corresponding plant for production of biogas from raw material comprising lignocellulose material, in particular from raw material with high amount of lignocellulose material in an energy and resource saving manner.

### Background of the Invention

Anaerobic digestion is the process by which bacteria and methanogenic archaea, living in an oxygen-free environment, convert organic matter into biogas.

Anaerobic digestion occurs naturally in environments rich in organic material and nutrients but low in oxygen, e.g. swamp marshes, lake beds, and in soil, but it can also be controlled in dedicated reactors, wherein biomass is fed to a live culture of microorganisms. These then produce biogas which is then harvested. Anaerobic Digestion plants, henceforth referred to as AD plants, utilize multiple reactors to generate biogas in large volumes, which can subsequently be processed for further commercial use.

AD plants were originally used as a waste management strategy for organic waste streams such as sewage sludge, manures, and food wastes to address odours, pathogens, and waste disposal, with renewable energy as a byproduct. In recent years, anaerobic digestion processes have been designed with a primary goal of generating renewable fuels and/or chemicals. To cover growing demand, alternative sources of biomass are needed.

Compared to traditional organic waste streams, lignocellulosic biomass shows promise as a feedstock for renewable fuel generation due to its abundance, added benefits to soil and water health, and provision of ecosystem services. Although lignocellulosic biomass is abundant, the lignin fraction forms a matrix around the cellulose and hemicellulose, providing protection from enzymatic hydrolysis and resulting in a feedstock that is recalcitrant to biological conversion.

Lignocellulosic biomass like wheat straw is comprised of 20-50% cellulose, 10-40 % hemicellulose, 10-30 % lignin, with the remaining percent being protein, lipids, and inorganic compounds. Cellulose and hemicellulose are both polysaccharides, i.e. organic polymers of sugars. In contrast, lignin is a group of organic polymers formed by cross-linked aromatic compounds which give structural support to cell walls. It is chemically very different to cellulose and hemicellulose as it generally resists reactions such as hydrolysis and oxidation which are otherwise key in the breakdown of organic matter. Lignin therefore poses a challenge to AD, not just because it itself is difficult to break down, but because in lignocellulosic material, lignin is in close contact with cellulose and hemicellulose and acts as a protective layer, hindering the AD. For the use of lignocellulosic biomass at an AD plant, it is therefore crucial that lignin is either broken down, such that the products can be converted to biogas, or processed in such a way that its presence does not hinder the AD of cellulose and hemicellulose. Conventional means of decomposing lignin prior to AD involve the pre-treatment of lignocellulosic biomass with either chemical, hydrothermal, or enzymatic processes. These options are often too expensive for commercial application. Alternatively, lignin may be removed as a waste product following the AD of cellulose and hemicellulose. The waste lignin can then be returned to the fields as fertiliser or burnt to generate process heat. Since lignin makes up 10-30 % of lignocellulosic biomass, only using it as fertiliser or fuel is wasteful and inefficient, so a method of converting lignin into biogas must be utilized.

The biological process of converting biomass into biogas is a multi-step process, but it can be summarized in four main steps: (Mulat and Horn., Biogas production from lignin via anaerobic digestion)

Hydrolysis: Biomass, which is generally comprised of cellulose, hemicellulose, lignin, proteins, and lipids, is broken down into its constituent parts: Cellulose and hemicellulose is broken down into mono- and oligomers of C6- and C5-sugars, respectively, protein is broken down into amino acids, and lipids are broken down into free short and long-chain fatty acids. This initial breakdown of the biomass is based on the reaction with water. This hydrolysis reaction is catalysed by enzymes which are released by hydrolytic bacteria into their surroundings.

Acidogenesis: This second step takes the products of the hydrolysis, i.e. sugars, amino acids etc., and further decomposes them into low molecular weight acids and alcohols by acidogenic bacteria.

Acetogenesis: The volatile fatty acids and alcohols are converted to acetate and hydrogen by hydrogen-producing acetogenic bacteria.

Methanogenesis: The formation of methane is done exclusively by methanogenic archaea. Methanogenic archaea are subdivided into two categories: Acetotrophic and hydrogenotrophic methanogens. Acetotrophic methanogens form methane by splitting acetate into carbon dioxide and methane, and hydrogenotrophic methanogens form methane by directly reducing carbon dioxide using formate or hydrogen as an electron donor. Both occur in the biogas reactor, thus utilizing all the acetate and hydrogen produced during acetogenesis.

Each step takes the products of the preceding step(s) and further decomposes them, such that the biological activity of each step is contingent on the biological activity of the preceding step(s). The biogas plant must therefore facilitate all steps to occur either simultaneously or sequentially.

For this biological process to occur, it is essential that key operating parameters are followed.
- Oxygen: Methanogenic archaea are strictly anaerobic, meaning the exclusion of oxygen (to low levels) is necessary for the formation of methane.
- Temperature: The reactors are held at elevated temperatures since biological and chemical processes proceed at higher rates. The choice of temperature also serves to promote certain biological processes by optimising living conditions for some microorganisms. Four temperature ranges are generally recognised in the context of AD plants. These are defined as: Psychrophillic (10-20 °C), mesophilic (20-45 °C), thermophilic (45-60 °C), and hyperthermophillic (60+ °C)
- Feeding: The rate and quantity of biomass fed to the biogas reactor is called the Organic Loading Rate (OLR). Pretreatment of the biomass may be beneficial to the rate of biogas production. This often includes milling the biomass into sufficiently small particles, such that hydration and subsequent AD is accelerated. It may also involve thermal, mechanical, or chemical procedures which decompose the biomass making it more susceptible to AD.
- Duration: The average time spent by the fluid entering a reactor is called the Hydraulic Retention Time (HRT). Removing material from a reactor also removes microorganisms. The rate of removal thus promotes the formation of certain groups of microorganisms.
- Nutrients: Beyond organic matter, the microorganisms also require other nutrients which are generally categorized into two categories. Macronutrients include phosphate, sulphate, and ammonia, i.e., compounds found in large concentrations. Micronutrients include elements such as iron, nickel, selenium, and tungsten. Depending on the choice of biomass, it may be necessary to supply nutrients to the reactors.

### Object of the Invention

The objective of the present disclosure is to achieve an efficient, energy and resource saving method and plant for producing a high-quality biogas from an organic raw material comprising at least 40% w/w lignocellulose material, such as straw and other available cellulosic feedstock.

### Short description of the invention

One objective of the present patent application is to provide a method for producing biogas from a biological raw material comprising at least 40% w/w lignocellulose material, the method comprising the steps of:
(a) providing an organic raw material comprising at least 40% lignocellulose biomass;
(b) forming a biomass slurry comprising the organic raw material and liquid comprising at least one of: fresh water and any of the different recycled streams available, such as part of the first permeate from the first fermentation step, part of the second permeate from the second fermentation step, part of the thick fraction from the liquid/solid separation step, a part of the third digestate from the third fermentation step, a part of the condensate from the evaporation step, a part of the first retentate from the first filtration step, and wherein process steps (b) and (c) can be two separate sequential process steps or one combined process step;
(c) subjecting the biomass slurry to a first fermentation process step, wherein the first fermentation is a hyperthermophillic anaerobic fermentation, thereby producing a first biogas stream and a first digestate;
(d) subjecting the first digestate to a second fermentation process step, wherein the second fermentation is a thermophilic anaerobic fermentation, thereby producing a second biogas stream and a second digestate);
(e) subjecting the second digestate to solid/liquid separation process, thereby producing a thin fraction and a thick fraction;
(f) recycling at least part of the thick fraction into the first fermentation step (c) and/or into the second fermentation step (d) and/or withdraw at least part of the tick fraction from the process;
   and either:
(g) optionally subjecting at least part of the thin fraction to a third fermentation process step, wherein the third fermentation is an anaerobic digestion, thereby producing a third biogas stream and a third digestate and wherein optionally a part of the third digestate is recycled into the first fermentation (c) and/or second fermentation (d) step, and/or bypass at least part of the thin fraction directly to the first filtration process step (h) thereby producing a first permeate and a first retentate;
(h) subjecting at least part of third digestate to a first filtration process step, thereby producing a first permeate and a first retentate;
(i) optionally subjecting at least part of the first retentate to a wet oxidation process step, thereby producing an oxidated first retentate and/or heat and/or inorganic byproduct, and/or withdraw at least part of the first retentate as a byproduct;
(j) when oxidated first retentate is produced, recycling at least part of the oxidated first retentate, into the first fermentation step (c) and/or into the third fermentation (g);
(k) subjecting at least part of the first permeate to a second filtration process step, thereby producing a second permeate and a second retentate;
(l) recycling at least part of the second permeate into the first fermentation (c); and
(o) collecting biogas from first, second and third fermentation steps (c, d, g) or:
(gg) bypassing process steps (g)-(n) by connecting the thin fraction stream and optionally a part of the thick fraction stream to another anaerobic digestion plant as additional lignocellulose biomass raw material; or by withdrawing the thin fraction and at least part of the thick fraction from the process; and
(oo) collecting biogas;
wherein the first fermentation step (c) takes place at temperature between 60-85 degree Celsius and the second fermentation step (d) takes place at temperature between 45-65 degree Celsius.

Another objective of the present disclosure is to provide a system or a plant for producing biogas from a biological raw material comprising at least 40% w/w lignocellulose biomass, wherein the system comprises:
- a first reactor, configured for digesting a biomass slurry comprising biological raw material comprising at least 40% w/w lignocellulose biomass mixed with liquid comprising at least one of: fresh water, and any of the different recycled streams available, such as part of the first permeate from the first fermentation step, part of the second permeate from the second fermentation step, part of the thick fraction from the liquid/solid separation step, a part of the third digestate from the third fermentation step, a part of the condensate from the evaporation step, a part of the first retentate from the first filtration step, by hyperthermophillic anaerobic fermentation, thereby producing a first biogas and a first digestate;
- a second reactor configured for receiving and anaerobically digesting the first digestate by thermophilic anaerobic fermentation, thereby producing a second biogas and a second digestate;
- a solid/liquid separator, configured for receiving and processing the second digestate and for producing a thick fraction and a thin fraction;
   and optionally:
- a third reactor configured for receiving and anaerobically digest the thin fraction by anaerobic digestion, thereby producing a third biogas (182) and a third digestate;
   and:
- a first filtration unit configured for receiving and filtering at least part of the third digestate, thereby producing a first permeate and a first retentate;
- a wet oxidation unit configured for receiving and oxidating at least part of the first retentate, thereby producing an oxidated first retentate;
- a second filtration unit, configured for receiving and filtering at least part of the first permeate, thereby producing a second permeate and a second retentate;
- a biogas collecting system, configured for collecting biogas from the first reactor and from the second reactor and optionally biogas from the third reactor; and
a recycling system for recycling over 50% of total liquid in the system, comprising means constructed for:
- Recycling stream comprising at least part of oxidated first retentate and/or at least part of first retentate into the first reactor and/or the third reactor;
- Recycling stream comprising at least part of third digestate and/or at least part of thick fraction into the first reactor and/or the second reactor;
- Recycling stream comprising at least part of recycling stream and/or at least part of second permeate stream comprising at least part of the condensate from the evaporation unit.

### Description of the drawings

Various examples are described hereinafter with reference to the figures. Like reference numerals refer to like elements throughout. Like elements will, thus, not be described in detail with respect to the description of each figure. It should also be noted that the figures are only intended to facilitate the description of the examples. They are not intended as an exhaustive description of the claimed invention or as a limitation on the scope of the claimed invention. In addition, an illustrated example need not have all the aspects or advantages shown. An aspect or an advantage described in conjunction with a particular example is not necessarily limited to that example and can be practiced in any other examples even if not so illustrated, or if not so explicitly described.

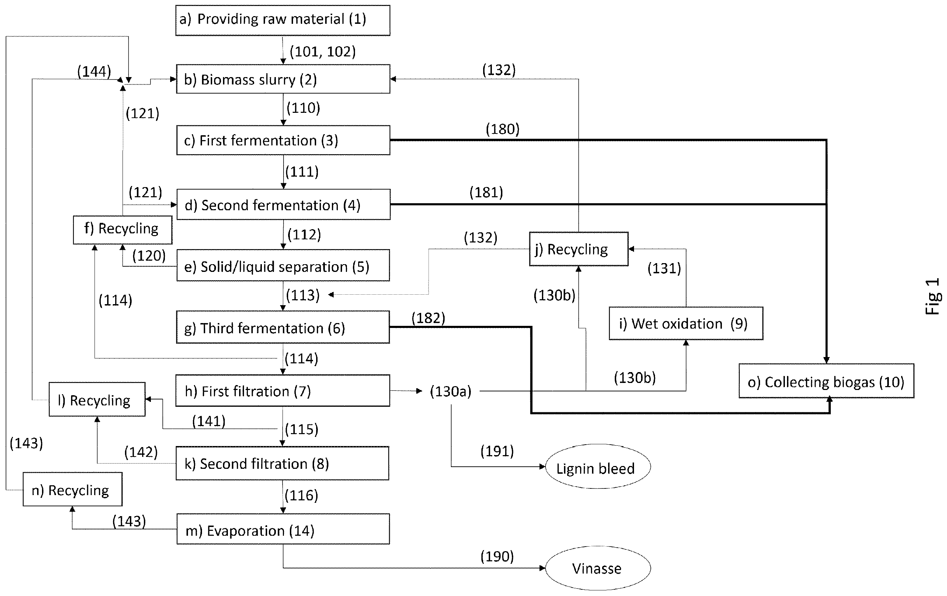

- Fig. 1: A flowchart illustrating a method for producing biogas from an organic raw material comprising at least 40% w/w lignocellulos according to the present invention.

In Fig. 1, a flowchart shows the process or method provided by the present invention for producing biogas from an organic raw material comprising at least 40% w/w lignocellulose biomass, where the different steps of the process are marked by reference letters a) to o), while different process streams, process units and products provided by the method are marked by number references.

**Table 1. Description of reference number or alphabetic letter in the drawings.**

| **No** | **Item's name and/or description** |
|---|---|
| 1 | Biomass management |
| 2 | Biomass slurry (Optional) |
| 3 | First reactor |
| 4 | Second reactor |
| 5 | Solid/liquid separator |
| 6 | Third reactor |
| 7 | First filtration unit |
| 8 | Second filtration unit |
| 9 | Wet oxidation unit |
| 10 | Collecting biogas / Central biogas processing system |
| 14 | Evaporation step / evaporation unit |
| 101 | Raw material |
| 102 | Fresh water |
| 110 | Combined material stream |
| 111 | First digestate from first fermentation unit |
| 112 | Second digestate from second fermentation unit |
| 113 | Thin fraction |
| 114 | Third digestate |
| 115 | First permeate from first filtration unit |
| 116 | Second retentate from second filtration unit |
| 120 | Thick fraction |
| 121 | Thick fraction and part of Third digestate |
| 130a | First retentate from first filtration unit |
| 130b | Another part of first retentate from first filtration unit |
| 131 | Oxidated first retentate |
| 132 | Recycled first retentate |
| 141 | Bypass of second filtration unit |
| 142 | Second permeate from second filtration unit |
| 143 | Condensate stream from the evaporation unit |
| 144 | Combined aqueous recirculation stream |
| 180 | Biogas from first fermentation unit |
| 181 | Biogas from second fermentation unit |
| 182 | Biogas from third fermentation unit |
| 190 | Concentrated vinasse stream from evaporation unit |
| 191 | Lignin rich byproduct |
| a | Providing biomass raw material |
| b | Creation of biomass slurry (Optional) |
| c | First fermentation |
| d | Second fermentation |
| e | Solid/liquid separation |
| f | First recycling step: |
| 9 | Third fermentation |
| h | First filtration |
| i | Wet oxidation |
| j | Second recycling step |
| k | Second filtration |
| l | Third recycling step |
| m | Evaporation (optional) |
| n | Forth recycling step (optional) |
| o | Collection of biogas |

### Detailed description of the Invention

Exemplary permutations will now be described more fully hereinafter with reference to the accompanying drawings. In this regard, the present examples may have different forms and should not be construed as being limited to the descriptions set forth herein. Accordingly, the examples are merely described below, by referring to the figures, to explain aspects.

Throughout the specification, when an element is referred to as being "connected" to another element, the element is "directly connected" to the other element, "electrically connected", "fluidic connected" or "communicatively connected" to the other element with one or more intervening elements interposed there between.

The terminology used herein is for the purpose of describing particular examples only and is not intended to be limiting. As used herein, the terms "comprises" "comprising" "includes" and/or "including" when used in this specification specify the presence of stated features, integers, steps, operations, elements, and/or components, but do not preclude the presence or addition of one or more other features, integers, steps, operations, elements, components, and/or groups thereof.

Unless otherwise defined, all terms used herein (including technical and scientific terms) have the same meaning as commonly understood by those skilled in the art to which this invention pertains. It will be further understood that terms, such as those defined in commonly used dictionaries, should be interpreted as having a meaning that is consistent with their meaning in the context of the relevant art and will not be interpreted in an idealized or overly formal sense unless expressly so defined in the present specification.

It should be noted that the method for producing biogas and the plant for carrying out the method, provided by the present invention are not limited for utilization for production of biogas. The method and plant provided by the present invention may also be used as an integral part of producing cellulosic biofuels including such fuels as bioethanol, biobutanol, and other fermentations fuels. Typically, such fuels are produced up-stream of the biogas production on cellulosic matter before fermentation residues are diverted to the method and/or plant provided by the present invention, or any other suitable anaerobic digestion (AD) plant.

In one embodiment of the present invention, the method and the plant are utilized for producing methane that is to be utilized as an interim product for up-stream production of different types of biofuels, such as bioethanol, biohydrogen, bio-jetfuel, biodiesel, bio-napthalene and other bioproducts including such products as biochemicals such as phenol, vanillin, styrene and several others. The list is not exhaustive.

In a possible embodiment, the biofuel in question is bioethanol and is produced upstream, or after the main production of biogas, such as by the method illustrated in Fig. 1., utilizing any suitable up-stream biogas processing methods and/or means. In one embodiment of the present invention, the biogas collected (180, 181, 182) is not the end product but rather an intermediate product to be upgraded to higher biofuels as for example jet fuel.

The tendency of lignocellulose material such as straw to form floating layers in a liquid solution, such as fermentation reactors, is well-known. The main reason for the floating layers forming on the surface of the liquid, is thought to be the wax layer enclosing the lignocellulose fibres, that prevents the penetration of moisture into the fibres. Prior art techniques have either utilized mechanical methods, which destroy or break up the floating layer with stirrers or jet stirrer devices, or by induced flow prevent formation of a floating layer.

WO 2013/152771 discloses method of pretreatment of lignocellulosic biomass raw material, where mechanical steam-explosions are utilized for repeated compressions of the biomass in a reciprocating piston press, resulting in a briquetted biomass. The briquetted biomass produced by the method of WO 2013/152771 demonstrates a water swelling factor or number of 5-10, meaning that the briquettes have exceptional water absorbing characteristics and are therefore well suited for using for the purpose of the present invention, as is any pretreated lignocellulosic biomass demonstrating similar characteristics.

Single feedstock fibrous lignocellulosic AD plants based on a conventional system design of low dry matter content input and high volumes of digestate are commercially prohibitory for the following reasons:
- Low biomass conversion: Anaerobic Digestion of lignocellulosic substrate requires high residence time, due to the large polymeric molecular structures, especially for lignin. Conventional system design AD plants struggle to achieve even 50% of the theoretical biogas yield (e.g. using straw as substrate the yield is normally below 250 NL CH4/kg VS).
- High water consumption: If lignocellulosic matter is to be the single feedstock, a conventional system design AD plant will require a minimum of 3-5 times the quantity of lignocellulosic matter in water to suspend and digest the matter.
- High nutrients consumption: Substantial quantities of nutrients must be added including macro-nutrients as ammonia, phosphate, and sulphate and micronutrients such as iron (Fe), manganese (Mn), zinc (Zn), cupper (Cu), cobalt (Co), nickel (Ni), molybdenum (Mo), selenium (Se), and tungsten (W). The list is not exhaustive. As an example, it is well described that the carbon to nitrogen ratio (C:N) should be no higher than 25-30 to enable optimal bacterial growth, however for wheat straw the C:N ratio is 70-100, necessitating the addition of nitrogen in the form of ammonia.
- Heat: For a conventional system design AD plant with a high throughput of substrate with a low dry matter content, a significant fraction of heat is lost via the disposal of digestate, even if heat exchange is applied.

Therefore, conventional system design AD plants are normally only capable of processing less than 20% fibrous lignocellulosic material as part of the substrate input.

The present invention provides a method and a plant for carrying out the method that is designed to tackle the obstacles described above. The overarching principles are:
- Optimize biomass conversion by means of membranes retaining undigested organic matter and thereby losing as little organic matter as possible to the digestate through recycling and accelerated (facilitated) biomass degradation.
- Reduce water consumption to a minimum by recycling. Consumption of water is thereby reduced to the amount required for the hydrolysis reactions and the water content selected in the digestate product (the fertilizer product is referred to as vinasse product/vinasse byproduct throughout this application).
- Reduce nutrients consumption to a minimum by recycling nutrients as much as possible while avoiding accumulating components to a level where they become inhibitory to the bacterial processes or in other ways causes difficulties for the plant.

As result, the method and the plant for carrying out the method, provided by the present invention, is commercially viable to use lignocellulosic biomass as a single feedstock for production of biogas. The process has been demonstrated at scale with a continuous surprisingly high gas yield (370-420 NL CH4/kg VS) despite a low total Hydraulic Residence Time (HRT) of less than 20 days, as demonstrated in the examples below.

The present invention thus provides a low-cost method and a plant for carrying out the method for using briquetted feedstock (according to the teachings of WO 2013/152771) or other suitable pretreated lignocellulose biomass raw material with similar water swelling or absorbing characteristics. The method and the plant provided by the present invention give rise to numerous advantages over prior art, including but not limited to:
- Significantly reduced capital cost for the pretreatment system and reduction of energy and water consumption during pre-treatment
- Considerable reduced tendency for floating straw layers, even without vigorous mechanical stirring in the reaction tanks utilized, resulting in significantly reduced tendency for processing bottlenecks at large scale
- Significantly reduced operating cost from pH adjustment
- Highly improved recovery of C5 sugars
- Significantly reduced toxicity of the soluble component of pretreated biomass against enzyme preparations
- Overall ethanol production per tons raw material can be increased by at least 10% simply due to conservation of feedstock through optimal feedstock logistics based on briquetting.

With reference to Fig. 1, the present invention provides a method for producing biogas from an organic raw material comprising at least 40% w/w lignocellulose biomass, the method comprising the steps of:
(a) providing a biological raw material (101) comprising at least 40% lignocellulose biomass;
(b) forming a biomass slurry (2, 110) comprising the biological raw material (101), water (102) and at least part of recycled streams (144, 121, 132);
(c) subjecting the biomass slurry (2) to a first fermentation process step, wherein the first fermentation is a hyperthermophillic anaerobic fermentation, thereby producing a first biogas stream (180) and a first digestate (111);
(d) subjecting the first digestate (111) to a second fermentation process step, wherein the second fermentation is a thermophilic anaerobic fermentation, thereby producing a second biogas stream (181) and a second digestate (112);
(e) subjecting the second digestate (112) to solid/liquid separation process, thereby producing a thin fraction (113) and a thick fraction (120);
(f) recycling at least part of the thick fraction (120) with or without a part of third digestate (114) into the first fermentation step (c) and/or into the second fermentation step (d);
(g) subjecting the thin fraction (113) to a third fermentation process step, wherein the third fermentation is an anaerobic digestion, thereby producing a third biogas stream (182) and a third digestate (114);
(h) subjecting at least part of third digestate (114) to a first filtration process step, thereby producing a first permeate (115) and a first retentate (130a);
(i) subjecting at least part of the first retentate (130a) to a wet oxidation process step, thereby producing an oxidated biomass (131) and/or heat and inorganic byproduct;
(j) when oxidated biomass (131) is produced, recycling at least part of oxidated biomass (131), into the first fermentation step (c) and/or into the thin fraction (113);
(k) subjecting at least part of the first permeate (115) to a second filtration process step, thereby producing a second permeate (142) and a second retentate (116);
(l) recycling at least part of the second permeate (142) into the first fermentation step (c);
(n) recycling the condensate stream (143) into the first fermentation step (c) and (o) collecting biogas (180, 181, 182) from first, second and third fermentation steps (c, d, g).

However, though not depicted in Fig. 1, it is possible to circumvent steps (g)-(n) in different ways. In one embodiment of the present invention, the stream of the thin fraction (113) and optionally a part of the thick fraction (120) stream are connected to a traditional anaerobic digestion (AD) plant as the lignocellulose biomass raw material for the AD plant. In another embodiment of the present invention, the thin fraction (113) and at least part of the thick fraction (120) from the system are withdrawn totally from the system and the thin fraction (113) is subjected to precipitation step and the supernatant is then recycled into the first fermentation step (c) and/or the second fermentation step (d) together with at least part of the thick fraction (120). It is understood that different combinations of bypassing approaches are possible, such as subjecting a part of the thin fraction to digestion in a traditional AD plant and/or recycle a part of the thin fraction (113) into the first fermentation (c) and/or second fermentation (d) and/or subjecting a part of the thin fraction (113) to a precipitation step and recycle the supernatant. If the thin fraction (113) is bypassed to another AD plant, it will have to be replaced by a different thin fraction (or another liquid stream with similar characteristics/material content) for the method and/or plant to function optimally.

### (a) Providing raw material:

As depicted by Fig. 1, the first process step (a) of the method provided by the present invention is to provide the raw material, by supplying lignocellulosic feedstock in the form of pressed briquettes of dry wheat straw, or other lignocellulosic feedstock exhibiting similar swelling characteristics as the pelleted lignocellulose biomass treated by the method disclosed in WO 2013/15277. In reference to Fig. 1, in the first process step (a) the raw material (101) comprising at least 40% w/w lignocellulose biomass is collected and pre-treated. It is understood that any known methods of collecting, storing, and pre-treating the lignocellulose biomass raw material (101) can be used.

### (b) Biomass slurry:

The second process step (b) of the method provided by the present invention is to create a biomass slurry comprising the biomass raw material (101) and liquid. This hydration of briquettes prior to fermentation accelerates incorporation of dry matter into the first fermentation reactor.

In the context of this application, the phrase liquid refers to all watery solutions or streams within the process or method for producing biogas and the plant or system for carrying out the method, as provided by the present invention. The terms total water and total liquid are used interchangeably within the context of this application, including when referring to the total water content of different streams, comprising varying amount of solids, such as the different recycling streams mentioned above (144, 132, 143, 121), and/or the total water/total liquid content of different reactors, such as the first reactor (3), second reactor (4), the solid/liquid separator (5), the third reactor (6) and the first (7) and second (8) filtration units and the wet oxidation unit (9).

In one embodiment of the present invention the liquid mixed with the biomass raw material (101) for creating the biomass slurry (2) comprises at least part of one or more of recycling streams (144, 143, 132, 121) while in another embodiment of the present invention the liquid mixed with the biomass raw material (101) for creating the biomass slurry (2) comprises fresh water (102). In yet another embodiment the biomass slurry is created by mixing biomass raw material (101) at least one of: fresh water (102) or at least part of at least one of the recycling streams (144, 132, 143, 121) or any combination thereof.

### (c) First Fermentation:

The third process step (c) of the method for producing biogas provided by the present invention is the first step of a chain of anaerobic fermentation process. Process step (c) is a hyperthermophillic anaerobic fermentation step, where mainly hydrolysis (biologically facilitated) takes place, whereby cellulose and hemicellulose from the lignocellulose biomass raw material reacts with water thereby breaking down into smaller molecules. This fermentation takes place at anaerobic hyperthermophilic conditions (60-80°C). This favours bacteria that enables hydrolysis, which is especially important for polymeric structures of cellulose and hemicellulose. The third process step (c), taking place in the first reactor (3) utilizes an operating temperature above 60 degrees Celsius, such as between 60-80 degree Celsius, such as between 65-75 degree Celsius, such as approximately 65-70 degree Celsius, such as about 65, 66, 67, 68, 69 or about 70 degree Celsius, such as 71, 72 73, 74 or about 75 degree Celsius. The retention time of the third process step (c) is between 1-5 days, such as between 2-4 days, such as 1, 2, 3, 4 or 5 days.

The hyperthermophillic anaerobic fermentation step of the third process step (c) takes place in the first reactor/hyperthermophillic fermenter/hyperthermophilllic digester (3). Any suitable Continuous Stirred Tank Reactor (CSTR) can be utilized. In one embodiment of the present invention, the plant for producing biogas provided comprises at least one first reactor (3), such as one or two or more first reactors (3) that operate in parallel, such as two, three, four or more first reactors (3) operating in parallel.

The third process step or first fermentation step (c) of the method for producing biogas from lignocellulose biomass, provided by the present invention, takes place in the first reactor (3). In one embodiment of the present invention the second process step (b), the creation of biomass slurry (2) takes place in a separate unit and a biomass slurry stream (110) is transported into the first reactor (3) while in in another embodiment of the present invention the biomass raw material (101) is mixed directly with liquid in the first reactor (3), whereby the second (b) and third (c) process steps take place simultaneously.

During the first fermentation step (3) of process step (c) there is an amount of biogas (180) being produced already in the first reactor (3). The first biogas (180) produced will rise to and above the liquid surface of the biomass slurry (2) in the upper section of the first reactor (3), where it is collected (o) as the first biogas stream (180).

As shown in Fig. 1, the first fermentation of third process step (c) further produces a first digestate (111), that is subjected to a second fermentation (d), in a second reactor (4), by transferring or pumping the first digestate (111) to the second reactor (4). During the first fermentation step (c), the first reactor (3) is kept stable, by ensuring that the input to the first reactor of organic raw material (101) and liquid comprising fresh water (102) and /or at least part of at least one of the recycling streams (144, 132, 143, 121, 120, 114, 142), that is the rate of filling of first reactor (3) is the same as the rate of removal of first digestate (111) from the first reactor (3).

### (d) Second fermentation:

The fourth process step (d) of the method for producing biogas provided by the present invention is the second step of a chain of anaerobic fermentation process. The second fermentation step (d) is a thermophilic fermentation that takes place in the second reactor (4). Any suitable reactor can be utilized for as the second reactor (4) for carrying on the second fermentation step (d). In one embodiment of the present invention, the plant for producing biogas provided comprises at least one second reactor (4), such as one or two or more second reactors (4) that operate in parallel. The first digestate (111) comprising the decomposed biomass from the third process step (c) is fed continuously to the second reactor (4) and fourth process step (d).

The fourth process step (d), taking place in the second reactor (4) utilizes an operating temperature above 45 degrees Celsius, such as between 45-65 degree Celsus, such as between 50-60 degree Celsius, such as approximately between 52-57 degree Celsius, such as about 50, 51, 52, 53, 54 or about 55 degree Celsius, such as 56, 57, 58, 59 or about 60 degree Celsius. The retention time of the second fermentation (d) is between 10 and 25 days, such as between 15-20 days, such as between 12-22 days, such as between 12-15 days, such as between 12-17 days, such as between 15-17 days, such as around 15, 16, 17, 18, 19 or 20 days.

In the second fermentation step (d) the main conversion of the above-mentioned smaller molecules as well as cellulose and hemicellulose into biogas takes place producing a considerable amount of biogas (181). Again, the biogas produced (181) will rise to, and above the liquid surface within the second reactor (4), into the upper section of the reactor, where it is collected (o) as the second biogas stream (181).

### (e) Solid/Liquid separation:

As shown in Fig. 1, the thermophilic fermentation (d) of the method for producing biogas provided by the present invention further produces a second digestate (112) that is subjected to a solid/liquid separation (e) in a solid/liquid separator (5), by transferring or pumping the second digestate (112) to the solid/liquid separator (5). The solid/liquid separation step (e) can by conducted by a screw press separator or any other suitable separator, where both size and/or number of the solid/liquid separators utilized will depend on the amount of second digestate (112). In one embodiment of the present invention, the plant for producing biogas provided comprises a screw press solid/liquid separator (5) capable of processing a second digestate stream (112) from more than one second reactor (4), while yet another embodiment of the present invention two or more solid/liquid separators (5) are utilized for processing second digestate stream (112) from two or more second reactors (4).

The fifth process step (e) divides the second digestate stream or streams (112) into two fractions or streams, a thick fraction (120) comprising a considerable part of the solid materials of second digestate (112) and a thin fraction (113) comprising considerable part of the liquid material of second digestate (112).

### (f) First recycling step:

In one embodiment of the method for producing biogas provided by the present invention, at least part of the thick fraction (120) produced in the fifth process step (e) is sent through a first recycling step (f) into the first fermentation step (c) and/or into the second fermentation step (d). The recycling of the thick fraction (120) as recycling stream (121) into the first (c) and/or the second fermentation (d) step/s ensures digestion/conversion of fibrous biomass.

The thick fraction (120) contains the vast part of the undigested lignocellulosic fibers above a certain size, that can be manipulated by changing the process parameters of the solid/liquid separator (5) for ensuring that the fibres that still comprise an important fraction of cellulose and hemicellulose that has not been converted yet are recycled for ensuring full digestion and conversion into biogas/other products. There is more than one way to manipulate the method and plant provided by the present invention, such as controlling the size of the fibres to be separated or the amount of dry-matter/non-converted organic compound to be separated into the thick fraction (120).

In one embodiment of the present invention the solid/liquid separation (4) or solid/liquid separator (5) is set to separate between 25%-75%, such as between 40-60%, such as around 50% of non-converted organic compound and recycle them into the thick fraction (120). In one embodiment of the present invention the solid/liquid separation is set to separate any undigested lignocellulosic fibres above 3mm. In another embodiment the solid/liquid separation is set to separate any undigested lignocellulosic fibers above 2 mm, such as above 3 mm, 3,5 mm or above 4 mm.

By adjusting different operational parameters of the Solid/liquid separation process (e) for distribution of dry and liquid matter, and by adjusting the distribution of destination for thick (120) and thin (113) fraction, the dry-matter content in the first (3) and second (4) reactors can be manipulated. Controlling the dry-matter content in the reactors (3, 5 and 6) is highly important. If the dry-matter content within the first reactor (3) is too high (>14%) then agitation becomes difficult requiring higher energy consumption, while for optimization the dry-matter content should be kept above 5%. As the second reactor (4) is in direct line to the first reactor (3), the dry-matter concentration is lower in the second reactor (4) than in the first reactor (3), due to the digestion or conversion that has already taken place in the first reactor (3). Manipulation of distribution of thick (120) and thin (113) fraction can therefore be utilized for controlling the dry-matter concentration in both the first and second reactor. If the dry-matter concentration in the first reactor (3) is considered too high, such as above 12%, more particularly above 14% or above 15%, the process can be adjusted by directing some of the thick fraction (120) back to the second reactor (4) or by adjusting the operation of the solid/liquid separator (5) parameters, for receiving a thick fraction (120) with lower dry-matter content.

In one embodiment only part of the thick fraction (120) produced in the fifth process step (e) is sent through a first recycling step (f) into the first fermentation step (c) and/or into the second fermentation step (d), while the remaining part of the thick fraction (120) is withdrawn from the method/plant.

As shown in Fig. 1, in one embodiment of the present invention, at least part of third digestate (114) (see discussion about first filtration (h) below) is recycled by the first recycling step (f) in into the recycling stream (121) of thick fraction (120) and into the first fermentation step (c) and/or the second fermentation step (d). The recycling of at least part of third digestate (114) enables partial conversion of the lignin content. This has been demonstrated through the process demonstration as depicted in the examples below.

### (g) Third fermentation:

As shown in Fig. 1, the thin fraction (113) produced in the solid/liquid separation step (e) is subjected to a third fermentation step (g), by transferring or pumping the thin fraction (113) to the third reactor (6). The sixth process step (g) or third fermentation step is an anaerobic digestion/fermentation.

In one embodiment of the present invention, the third reactor (6) is a fixed-film/biofilm reactor, other reactors, suitable for anaerobic digestion/fermentation can be utilized for the sixth process step (g) of the present invention.

As shown in Fig. 1, the sixth process step (g) of the present invention produces a third digestate (114) and a third biogas stream (182), that is collected (o) with the other biogas streams (180 and 181).

In one embodiment of the present invention, the plant for producing biogas comprises a biofilm or a fixed-film reactor acting as the third reactor (6), wherein the third reactor (6) comprises a tank comprising a mesh/grid comprising immobilized bacteria cultures. In one embodiment provided by the present invention the third fermentation step (g) utilizes a fixed-film reactor and digests small fibers and Volatile Fatty Acids (VFAs) present in the thin fraction of the second digestate (112) in an effective and robust manner, thereby rendering the third digestate in a state that can be separated in the first filtration.

It has surprisingly been discovered that by utilizing a fixed film/biofilm fermenter/reactor operated at thermophilic temperatures as a down-stream treatment of effluent from a thermophilic upstream digestion can serve as yet another direct recipient of treated lignin without impairing the digestion.

Bacteria and archaea engaged in methanogenesis during anaerobic processes potentially build biofilms by adhering or attaching to biofilm carriers. It is known in the state of art that the efficiency of anaerobic digestion may be increased by promoting microbial retention through biofilm development. The inclusion of certain types of biofilm carriers may differentiate existing AD biofilm reactors through their respective mode of biofilm growth, and meta-analyzed results of the state of art have depicted varying degrees of biogas enhancement. Furthermore, different carrier materials highly promote the dynamics of the dominant microbial population in each system.

Our observation of unimpaired digestion and even highly efficient digestion of treated lignin suggests a surprisingly large degree of robustness against the toxicity of compounds formed during lignin treatment.

Even more surprisingly, it has been discovered that not only dissolved matter is digested almost completely, but even suspended lignin colloids are partly digested during biofilm treatment. We interpret these phenomena as a result of the promotion of surface contact and improvement of interspecies electron transport. It is the biofilm's composition and structural organization being responsible for the added function of the special layer of microbial cells.

### (h) First filtration step:

In one embodiment of the present invention, at least part of third digestate (114) is subjected to a first filtration step (h) by transferring or pumping at least part of third digestate (114) to the first filtration unit (7), (see discussion about first recycling step (f) for the possible recycling of at least part of third digestate (114)), thereby producing a first permeate (115) and a first retentate (130a).

In one embodiment of the present invention, the plant for producing biogas comprises an ultrafiltration membrane filtration (UF) acting as the first filtration unit (7). The ultrafiltration membrane filtration (UF) unit is a filtration unit that utilizes a low-pressure membrane filtration process. Other filtration units or filtration methods suitable for separating any macromolecules and particles including those comprised of suspended solids, bacteria, colloidal matter and proteins from the liquid, in the third digestate (114) can be utilized for the seventh process step or first filtration (h) of the present invention. In another embodiment of the present invention, first filtration unit (7) comprises pre-filtering means and ultrafiltration membrane filtration (UF) unit. In one embodiment of the present invention, the first filtration step of the method comprises an ultra-membrane filtration step and/or one or more pre-filtering steps.

The filtration of at least part of third digestate (114) is an essential process step for limiting the accumulation of compounds that can interfere with the anaerobic digestion steps.

It is possible to adjust the two different streams possible for third digestate (114), thereby controlling the ratio of third digestate (114) that is recycled and/or subjected to the first filtration step (h) and/or withdrawn as a lignin bleed (191). It is possible to manipulate and optimize the method and the plant provided by the present invention by adjusting the process parameters of the first filtration step (h), where it is beneficial to reach a level of recycling of third digestate (114) where as much as possible is recycled, while ensuring that the levels of different ions and compound, such as Si and Ca and lignin are kept at save level. It should therefore be noted that different ratios of division of the third digestate (114) between the four possible streams are all considered within the scope of this invention, such as dividing the third digestate (114) into four streams to first fermentation step (c), second fermentation step (d), first filtration (h) and out of the system as a lignin bleed (191), or subjecting the whole of third digestate (114) to a first filtration step (h). In one embodiment the third digestate (114) is divided into two streams of recycling into first fermentation step (c), second fermentation step (d) and drawn out of the system as a lignin bleed (191), in another embodiment the third digestate is divided into two recycling streams into first fermentation step (c) and second fermentation step (d) and a third stream into the first filtration step (h

### (i) Wet oxidation:

As demonstrated in Fig. 1, the first filtration step (h) produces a first permeate (115) and a first retentate (130a), wherein the first retentate (130a) comprises a high concentration of lignin, and as shown in Fig. 1, it is possible to withdraw from the method/plant, at least part of the first retentate (130a) as a separate lignin bleed (190).

In one embodiment of the present invention, at least part of the first retentate (130a) is subjected to an oxidation step (i) as part-stream (130b), thereby producing an oxidated biomass (131) and/or heat and inorganic byproduct. The purpose of the wet oxidation step (i) is to open some of the aromatic rings of the lignin structure by oxidization and hydrolysis to leave a larger part of the lignin molecules more accessible to biogas digestion bacteria when it is circulated back to the digestors, thereby enabling acceleration of the degradation of the "hard to digest" components of lignocellulose, especially lignin. In another embodiment of the present invention, at least part of the first retentate (130a) is withdrawn from the method and/or plant without subjecting it to wet oxidation.

Another beneficial effect of applying the wet oxidation step (i) is the generation of heat, that can be utilized in the plant. In one embodiment of the present invention the wet oxidation step (i) produces only heat and inorganic byproduct, in another embodiment of the invention the wet oxidation step (i) produces oxidated biomass (131) and heat and organic byproduct, while in yet another embodiment of the present invention the wet oxidation step produces oxidated biomass (131) without producing any inorganic byproduct.

It has surprisingly been discovered that partially wet oxidized lignin does not inhibit the microbial activities during the hyperthermophilic digestion nor the following thermophilic digestion. The explanation is most likely that the mainly hydrolytic and fermentative bacteria in this hyperthermophilic digestion are capable of hydrolysing, fermenting and partly digesting the inhibitory components produced during wet oxidation of the lignin. Such initial destruction of toxins protects subsequent methanogenic activity of the following AD step.

The state of art may inter alia be referenced by the quote below (Wet explosion of wheat straw and codigestion with swine manure: Effect on the methane productivity. G. Wanga et al., Waste Management 29 (2009) 2830-2835):
"Despite the high release of soluble sugars (Fig. 2a) the methane obtained from the *wet-exploded wheat straw was slightly lower compared to the raw biomass (232* ± *13 and 244* ± *11 compared to 261* ± *1 ml CH4 perg TS, respectively). This, most probably, was due to the formation of inhibitory compounds, which is a very common problem when pretreatment at elevated temperatures is employed prior to fermentations (Klinke et al., 2004). The same phenomenon was reported by Penaud et al. (2000) where the methane production declined after thermochemical pretreatment although the biomass was solubilized at a high degree. In the study of Eskicioglou et al. (2007) inhibition of methane production was also observed after microwave pretreatment of waste activated sludge but subsequent acclimation of anaerobic biomass was possible and finally solubilisation of the substrate led to increased methane production* as *well. Inhibitors formed during pretreatment of lignocellulosic biomass at high temperatures and acidic conditions, i.e. furfural, 5-hydroxymethylfurfural and phenolic compounds, is also* a *commonly known fact (Palmqvist and Hahn-Hagerdal, 2000). Taking into consideration the low differences between methane potential values and the standard deviation we can safely conclude that the wet explosion pretreatment did not have any significant effect on methane production. In fact, wet explosion resulted in a slight reduction of methane production.*

*In any case, wet explosion (which is an energy consuming process) is not suitable* as *pretreatment of straw for codigestion with manure at the conditions tested because the additional required energy consumption is not counterbalanced by any increase in methane production."*

Klinke, H.B., Thomsen, A.B., Ahring, B.K., 2004. Inhibition of ethanol-producing yeast and bacteria by degradation products produced during pre-treatment of biomass. Applied Microbiology and Biotechnology 66, 10-26.

Penaud, V., Delgenes, J.P., Moletta, R., 2000. Influence of thermochemical pretreatment conditions on solubilisation and anaerobic biodegradability of a microbial biomass. Environmental Technology 21 (1), 87-96.

Eskicioglou, C., Terzian, N., Kennedy, K.J., Droste, R.L., Hamoda, M., 2007. Athermal microwave effects for enhancing digestibility of waste activated sludge. Water Research 41 (11), 2457-2466.

Palmqvist, E., Hahn-Hägerdal, B., 2000. Fermentation of lignocellulosic hydrolysates. II: inhibitors and mechanisms of inhibition. Bioresource Technology 74, 25-33.

Khan and Ahring, 2019, Lignin degradation under anaerobic digestion: influence of lignin modifications - A review, Biomass and Bioenergy, 128

Toda et al, 2017. Inhibition of anaerobic digestion by dissolved lignin derived from alkaline pre-treatment of an aquatic macrophyte, Chemical Engineering Journal, 311.

Further consideration of inhibition by lignin products from wet oxidation are given in Mulat and Horn (2018) They suggested two strategies (acclimation/adaptation and co-digestion) to be implemented for the efficient and stable operation of lignin-rich biogas plants. Notwithstanding these strategies we found surprisingly effective digestion of inhibitory compounds by our three tiers system including the hyper thermophilic, thermophilic and thermophilic biofilm digestion.

### (j) Second recycling step:

As portrayed in Fig. 1, any oxidated biomass (131) produced by the wet oxidation step (i) is subjected to the second recycling step (i) and returned to the first (c) and/or third (g) fermentation step, for enabling further anaerobic digestion of the contents of the oxidated biomass (131) produced by the wet oxidation step (i). By utilizing recycling steps, the microbiological matter/bacteria contained in the effluents (digestates) from previous fermentation stages is returned (in partially dissolved and disintegrated form), thereby returning both nutrients and carbon from the bacteria to the fermentation processes.

### (k) Second filtration:

As depicted in Fig. 1, the first filtration step (h) of the present invention produces a first permeate (115) and a first retentate (130a, 130b). At least part of the first permeate (115) is subjected to a second filtration step (k) by transferring or pumping the first permeate (115) to the second filtration unit (8), thereby producing a second permeate (142) and a second retentate (116).

The purpose of the second filtration step (k) is to produce the second permeate (142), that is at least partly subjected to the third recycling step and thereby recycled into the biomass slurry (b)/first fermentation (c). By subjecting at least part of the first permeate (115) to the second filtration (k) a second permeate (142) is produced that comprises limited amount of solids and salts/ions, and it is thereby ensured that continuous recycling of second permeate (142) does not build up the concentration of salts/ions to an undesired level within the plant provided by the invention.

The present invention provides a method and a plant for carrying on the method, that comprises many adjustable process parameters. Yet another process parameter that is adjustable, is the ratio of first permeate (115) that is subjected to a second filtration step (k), and thereby it is possible to control or manage the buildup of salts/ions within the plant. In one embodiment of the present invention, the whole of first permeate (115) is subjected to a second filtration step (k). In another embodiment of the invention the whole of first permeate (115) is recycled as recycling stream (141) into the third recycling step (l) and into the biomass slurry (b)/first fermentation (c). It is furthermore possible to adjust the amount of the second permeate (142) that is recycled (I), as part of the second permeate (142) can be withdrawn from the method/plant.

In one embodiment of the present invention, the filtration unit (8) utilized for the second filtration step (k) is a reverse osmosis (RO) filtration unit.

### (l) Third recycling step:

As explained above in the discussion of the first (h) and second (k) filtration steps, the third recycling step is the recycling of at least part (141) of first permeate (115) and at least part of the second permeate (142) into the first fermentation step (c). The third recycling step (l) ensures water recycling and thereby contributes to reduced water consumption of the method and plant provided by the invention.

As previously mentioned, the present invention provides a method for producing biogas and a plant for carrying on the method that comprises many adjustable process parameters. Manipulating the distribution between (142) and (141), including the amount/ratio of the first permeate (115) that is recycled as recycling stream (141) and how much of the second permeate (142) is recycled and how much of the second permeate (142) is drawn from the method/plant enables control of the concentration of nutrients and other components in the recycled water.

### (m) Evaporation:

As shown in Fig. 1, at least part of the second retentate (116) produced by the second filtration step (k) is subjected to an evaporation process step (m) in an evaporation unit (14), thereby producing a concentrated vinasse stream (190) and a condensate stream (143).

The purpose of the evaporation step (m) is to further process the second retentate (116), that comprises high concentrations of minerals. The second retentate (116) can in principle be considered a biofertilizer, however the second retentate (116) still contains a large percentage of water, that can be beneficial to remove for storage and transportation consideration.

The process parameters of the evaporation step (m) can be manipulated as to achieve a fluid vinasse stream (190) with different dry matter content.

In one embodiment of the present invention, the evaporation unit (14) utilized is a Mechanical Vapor Recompression (MVR) unit, that is a proven energy-saving evaporative concentration technology, which reduces evaporation energy use by 90% or more. MVR uses energy recovered from the condensate to evaporate further liquid. Any other suitable evaporation unit can be utilized.

### (n) Fourth recycling step:

As deposited in Fig. 1, the evaporation process step (m) in an evaporation unit (14), produces a concentrated vinasse stream (190) and a condensate stream (143), wherein the condensate stream (143) comprises evaporated water that can be recycled by the fourth recycling step (n) into the first fermentation step (c) for further reducing the water consumption.

It should be noted that that even though not specifically depicted in Fig. 1, any condensate from the biogas collection step (o) can be recycled.

As discussed above conventional single feedstock cellulosic AD plants are commercially prohibitory especially due to low extent of conversion of biomass as well as the requirement of Water, Nutrients and Heat.

The requirement of nutrients to allow for single lignocellulosic feedstock have been reported and discussed in literature, e.g. Scherer et al., Bioengineering 2022, 9(1), 13.

The necessary addition of water and nutrients are costly both regarding the direct acquisition, but also indirectly due to the disposal of a large quantity of liquid digestate. Even if the nutrients are partly returned as fertilizer via the digestate and the value partly recovered, the outcome is commercially prohibitive.

The present invention provides a method of producing biogas utilizing single feedstock cellulosic raw material and a single feedstock cellulosic AD plant for carrying out the method, that alleviate the above-mentioned constraint, especially due to the application of the recycling steps provided by the present invention.

In the present application, total liquid, in the contest of "total liquid" within the plant or "total liquid" within the system or "total liquid" utilized by the method provided, or in any other content, is to be recognized as the total amount of liquid processed by the method and/or the plant. This includes any fresh water added anywhere within the plant/method and all liquid comprised within the utilized raw materials. Obviously, the total liquid will greatly depend on the raw materials utilized. As explained above, the present invention provides a method and a plant for carrying out the method for producing biogas from organic raw material comprising at least 40% w/w lignocellulose biomass. However, the amount or % of w/w lignocellulose biomass utilized will both influence the amount of liquid that comes into the system by the raw materials utilized and the possible recycling of liquid. When the raw material utilized comprise just above 40% w/w lignocellulose biomass, the rest of the biomass supplied will probably comprise considerably more liquid, classic example is to utilize manure for AD plants.

However, present invention provides a method and a plant for carrying out the method for producing biogas from organic raw material comprising considerably higher % of lignocellulose biomass, such as over 80%, over 90%; over 95% and close to 100% lignocellulose biomass. In one embodiment the present invention provides a method and a plant for carrying out the method for producing biogas from organic raw material comprising a 100% straw-based lignocellulose biomass or between 98-100%, where op to 95% of total liquid is recycled, such as between 90-95%, such as between 92-94%, such as 93-94%, such as above 90% of total liquid is recycled or recirculated. This large-scale liquid recirculation provides a recycling loop, where the liquid can be recycled op to 20 times, such as between 10-20 times, more than 10 times, such as between 12-17 times, such as up to 15 times, depending on the organic biomass raw material used. The amount of recirculation depends on the amount of total liquid provided by the organic raw material, such as if the organic raw material comprises 40% of lignocellulose biomass, the recycling loop will be limited to below 10 times, such as below 5 times.

It is therefore one of the many surprising beneficial effects of the present invention that the method and plant for carrying on the method for producing biogas, is capable of not only utilizing extremely high w/w% lignocellulose biomass, but also to limit the water consumption considerably compared to prior art AD plants and methods.

The present invention provides a method of producing biogas utilizing single feedstock cellulosic raw material and a single feedstock cellulosic AD plant for carrying out the method, comprising different options and process parameters regarding treatment of the highly concentrated lignin stream of the first retentate (130a). When the first retentate (130a) is subjected to the wet oxidation (i), different flexible and adjustable process parameters arise, as it is possible to manage the recycling of heat, water and nutrients, and even further the option to digest any and all of the lignin residues.

### First option: Utilizing the second recycling step (j) for recycling oxidated first retentate (131) to first fermentation (c).

As previously described, we extract the first retentate (130a) comprising high concentrations of lignin by applying the first filtration step (h), whereafter at least part of the first retentate (130a) is subjected to a wet oxidation step (i) by means of a partial oxidation under a specific set of parameters producing an oxidated first retentate (131).

The oxidated first retentate (131) comprises a slurry comprising 5-30% dry matter comprising high concentration of lignin, wherein in the range of 10-50% of the lignin is completely converted, i.e., oxidized to heat and CO2, while the remaining lignin appears as carboxylic acids, mono- and oligomers of aromatic lignin constituents, and other lignin fragments.

The treatment parameters applied in the wet oxidation process step (i) are such that temperature is in the range 100-200 degree Celsius, such as between 120-180 degree Celsius, such as between 140-160 degree Celsius, such as around 140 degree Celsius or around 150 degree Celsius or around 160 degree Celsius, pressure is in the range 20 - 200 bar such as between 50-150 bar, such as between 80-120 bar, such as around 80, 90, 100, 110 or 120 bar, retention time is in the range 1-30 minutes such as between 1-20 min, such as between 5-15 min, such as around 7, 8, 9, 10, 11, 12 or 13 minutes. The resultant treated liquid is hot.

Furthermore, oxidation of lignin (i) is an exothermic process and thus heat is generated. The present invention provides a method of producing biogas utilizing single feedstock cellulosic raw material and a single feedstock cellulosic AD plant for carrying out the method, wherein the heat from full and/or partial wet oxidation (i) can be utilized for:
I) pre-heating the first retentate (130b) stream before subjecting it to the wet oxidation step (i)
II) applying the heat for heating the initial hyper-thermophilic fermentation of the first process step (c)
III) further harvesting of heat e.g. via steam or hot-water generation to be used elsewhere throughout the process/plant.

Thus, heat is recycled.

As previously described, the first retentate (130a) also contains microorganisms as well as dissolved and suspended macro- and micronutrients. In particular, the essential micronutrients immobilized by the microorganisms, including the rare metals such as nickel and tungsten, will be unlocked and can therefore be reutilized.

Thus, nutrients are recycled.

The present invention provides a method of producing biogas utilizing single feedstock cellulosic raw material and a single feedstock cellulosic AD plant for carrying out the method wherein the initial hyperthermophilic digestion of the first fermentation step (c) receives the brunt of the organic loading and the brunt of the characteristics of the cellulosic matter including the low content of nutrients. Also, at this stage heat is required alongside the nutrients. It is therefore highly efficient to recycle heat, water and nutrients to this particular stage, the first fermentation (c) of the 3-step AD process of the present invention.

### Second option: Utilizing the second recycling step (i) for recycling oxidated first retentate (131) to the third fermentation (g).

The second option is to recycle oxidated first retentate (131) to the third fermentation step (g). The thermophilic biofilm digester (6) allows for all functional groups of microbial processes to coexist on a biofilm and therefore to perform all main functions of the AD process, including hydrolysis, fermentation, acetogenesis, and methanogenesis.

When oxidated first retentate (131) is recycled to the third and final fermentation step (g), we are able to digest the inhibitory components as well as dissolved matter, including carboxylic acids and aromatic mono- and oligomers. As mentioned previously, we even digest a fraction of the suspended lignin particles.

The recycling of oxidated first retentate (131) to the third and final fermentation step (g), i.e., down-stream of the more sensitive methanogenic digestion of the second fermentation (d) step, thereby protecting the more sensitive methanogenic process of the second fermentation (d), while any possible undecomposed matter remaining after the third fermentation (g) will be concentrated into a first retentate (130a) by the first filtration (h) unit and treated again in the oxidation unit (9).

As described above, the present invention provides a method of producing biogas utilizing single feedstock cellulosic raw material and a single feedstock cellulosic AD plant for carrying out the method, wherein further permutations of the technology are possible in that not only oxidated first retentate (131) may be recycled but also untreated third digestate (114) produced by the third fermentation (g), thereby bypassing the first filtration unit (7). This will reduce the volume of liquid to be subjected to the first filtration (h) and volume that has to be treated in the first filtration unit (7), thereby increasing the concentration of lignin in the first retentates (130a, 130b) while at the same time reducing the total quantity to be extracted by the first filtration unit (7). The latter is due to a greater fraction of the lignin being digested by the microorganisms without prior treatment.

Also, the oxidated first retentate (131) may be recycled to second fermentation step (d) (not depicted in Fig. 1) in measured and controlled quantities to supply the second reactor (4) with heat, nutrients, and water via the oxidated first retentate (131).

By and large, one permutation allows for recycling to all three fermentation steps in measured and controlled quantities.

### (o) collecting biogas:

As shown in Fig. 1, and discussed above, the biogas stream (180) produced by the first fermentation step (c), the biogas (181) stream produced by the second fermentation (d) and the biogas stream (182) produced by the third fermentation step (g) are all collected by the collecting biogas step (o).

Further process steps for storing and treating the collected biogas are possible, though not depicted in Fig. 1.

### Examples

Below are results from semi-scale tests from a semi-scale test facility that has been in operation since the beginning of 2022 with briquetted wheat straw as the feedstock, while initially using a liquid fraction of the digestate from a manure-based biogas plant as the nutrient source during the startup phase. Since June 21st 2023, the facility has been operated completely isolated with briquetted wheat straw as the single feedstock without the continuous addition external of nutrients. Thus, the nutrients contained in the system (needed for the fermentation processes) have been recycled continuously. Only in the case where a deficit in certain nutrients was detected have these been added specifically.

Highlights of the semi scale test facility:
- First Fermentation: 10m3 CSTR
- Second Fermentation: 30m3 CSTR
- Third Fermentation: 7 m3 biofilm reactor
- First Filtration: Ultra-filtration including pre-filters up to 1m3/h
- Continuous process monitoring and control
- Gas production monitored by Drum-Type Volumetric Gas Meters

### Gas yield

The gas yield has been calculated from data and measurements according to the following formula: Volume of methane produced / mass of fresh organic substance added [NL CH4/kg VS]

Volume of methane produced, derived by:
- The actual biogas volume is measured by Drum-Type Volumetric Gas Meters providing pulses which are recorded by a computer which allows monitoring the daily production of biogas from the 3 fermentation stages. Furthermore, the temperature of the biogas at the gas meter is measured and the pressure is known from the process monitoring.
- This normalized biogas volume reported in Normal Liters (@0°C and 1atm) is calculated by:
   Normalized biogas volume =
   Actual measured volume from volumetric gas meter x (gauge pressure in digester in mBar + 1,01035) / 1,01035 x (measured gas temperature in °C +273,15) / 273,15
   x (1 - x(H2O)%)
   where x(H2O)% is the water vapor content (in mole%) in biogas at the measured temperature and pressure.
- The methane content in biogas is measured (along with a number of other components) on regular basis using a calibrated instrument. The sum of CH4 and CO2 is calculated and used for normalization, so that CH4 + CO2 = 100%. The daily average methane concentration in the biogas is calculated from this data and multiplied by the Normalized biogas volume to derive Volume of methane produced from each fermentation stage.

Mass of fresh organic substance (VS) added:
- The amount of fresh straw added to the system per day is recorded on a scale.
- The organic substance added, is determined by applying the VS content of the straw which is measured on a regular basis, by the standard methods of determining DM (oven @105°C 24h) and VS (oven @550°C 1h). In between dates of measurement, linear interpolation is applied.
- The daily gas production is not just a result the amount of VS added on the day or day before, but rather an average of a longer period before. Therefore, in the calculation of gas yield a weighted average of three weeks is applied.

The calculated gas yield for fermentation stage 1 and fermentation stage 1 and 2 together since the middle of June 2023 is shown in table 2., that shows gas yield, dry Nm3 CH4/ton VS; first (c) and second (d) fermentation yield, from a plant according to the present invention comprising the three fermentation steps (c, d and g) of the present invention, solid/liquid separation (e) step and a first filtration step (h). Gas yield is corrected for gas temperature, pressure and moisture.

It should be noted that during this period, matter from the wet oxidation process has not yet been recycled to the system. Thus, the gas yield reported is achieved entirely through the fermentation processes combined with some of the claimed recycles of the process system.

**Table 2. Gas yield from a semi-scale test facility**

| **Periodical averages** | | |
|---|---|---|
| From | To | Gas yield |
| 01-01-2023 | 19-03-2023 | 175,3 |
| 20-03-2023 | 19-05-2023 | 187,6 |
| 20-05-2023 | 19-06-2023 | 219,7 |
| 20-06-2023 | 27-06-2023 | 264,5 |
| 28-06-2023 | 05-07-2023 | 266,0 |
| 06-07-2023 | 13-07-2023 | 301,7 |
| 14-07-2023 | 21-07-2023 | 402,1 |
| 22-07-2023 | 29-07-2023 | 464,9 |
| 30-07-2023 | 06-08-2023 | 455,1 |
| 07-08-2023 | 14-08-2023 | 406,8 |
| 15-08-2023 | 22-08-2023 | 440,8 |
| 23-08-2023 | 30-08-2023 | 439,3 |
| 31-08-2023 | 07-09-2023 | 377,7 |
| 08-09-2023 | 15-09-2023 | 407,8 |
| 16-09-2023 | 23-09-2023 | 390,2 |
| 24-09-2023 | 01-10-2023 | 371,9 |
| 02-10-2023 | 09-10-2023 | 374,2 |
| 10-10-2023 | 17-10-2023 | 388,1 |
| 18-10-2023 | 25-10-2023 | 417,8 |
| 26-10-2023 | 02-11-2023 | 377,3 |
| 03-11-2023 | 10-11-2023 | 380,4 |

As can be seen, the gas yield is at a high level for a period of more than 3 months. Since the beginning of September 2023, the gas yield has stabilized in range of 370-420 NL CH4/kg VS. This documents that even without the application of wet oxidation, the claimed process system design is able to convert other constituents of lignocellulose in addition to cellulose and hemicellulose contents.

This can be concluded from the fact that typically around 75% of the organic content in wheat straw is cellulose and hemicellulose combined (REFERENCE to Phyllis database). Complete conversion stoichiometric of cellulose and hemicellulose yields 413 and 423 NL CH4/kg VS respectively. Thus, complete conversion of all cellulose and hemicellulose content should not result in total gas yields of more than 330 NL CH4/kg VS even if the added straw has unusually high contents of cellulose and hemicellulose.

### UF permeate and concentrate analysis:

**Table 3: Nutrient Separation During Ultra Filtration**

| **Nutrient Separation During Ultra Filtration** | | | |
|---|---|---|---|
| **Component** | **Permeate** | **Concentrate** | **Unit** |
| Total Dissolved Solids | 5,57 | 31,53 | g/l |
| Suspended Solids | 32 | 18000 | mg/l |
| Ammonia/ammonium (NH3/NH4+) | 550 | 650 | mg/l |
| Kjeldahl-N | 590 | 1200 | mg/l |
| Orthophosphate (PO43-) | 4,6 | 70 | mg/l |
| Sulphate (SO42-) | 6,4 | 6 | mg/l |
| Chloride (CI-) | 800 | 810 | mg/l |
| Sodium (Na) | 270 | 310 | mg/l |
| Potassium (K) | 1900 | 2300 | mg/l |
| Magnesium (Mg) | 94 | 440 | mg/l |
| Calcium (Ca) | 62 | 1800 | mg/l |
| Manganese (Mn) | 0,051 | 22 | mg/l |
| Iron (Fe) | 2,5 | 120 | mg/l |
| Cobalt (Co) | 2,1 | 61 | µg/l |
| Nickel (Ni) | 23 | 330 | µg/l |
| Copper (Cu) | 48 | 3800 | µg/l |
| Zink (Zn) | 120 | 15000 | µg/l |
| Selenium (Se) | 1,7 | 50 | µg/l |
| Tungsten (W) | 6,3 | 14 | µg/l |

We have found that the ultra filtration separates nutrients in a way that allows for their efficient recycling. Unwanted components, notably sodium, potassium, and chloride pass the UF filter unaffected, after which they may be upconcentrated during the second filtration (reverse osmosis, step (k)) and eventually isolated after evaporation (step (m)). Macronutrients such as phosphate, nitrogen compounds (Kjeldahl-N), and micronutrients such as iron, nickel, tungsten etc. are found in the UF concentrate. These compounds are likely bound in either an organic matrix of lignin fragments or in microbial cell residues, which are also removed during UF. The wet oxidation of the concentrate breaks down the organic material, leaving the nutrients available for re-digestion once they are injected into an earlier step in the fermentation process, as discussed previously.

For successful manipulation and optimization of the method and the plant of the present invention, various optional measurements of nutrients and toxins at key points can be applied for determining optimal recycling procedures, as well as for determining the potential necessity for adding nutrients, thereby reducing running costs and resulting in a more stable AD plant.

### In conclusion:

The surprising discoveries discussed above greatly affect the efficiency and functionality of the entire new systems solution being the invention as presented here.

The innovation provides a breakthrough in single feedstock and co-digestion feedstock lignocellulosic AD plants. It does so by providing for almost 100% digestion of the organic matter added to the plant.

It ensures through recycling of water that no large quantity of liquid digestate is produced, but only the biogas and the concentrated minerals appear as the sum total of outputs.

The consumption of water, nutrients and other consumables as heat and power is minimal relative to the output.

The hall marks of the Method & Plant are a highly effective biogas process with a minimal use of consumables achieved by:
Recycling of any and all nutrients as required
- Recycling of water as required
- Recycling of heat as required
- Potential generation of heat as required through wet oxidation
- Elimination of toxicity of treated lignin to the AD process thus ensuring unimpaired anaerobic digestion
- A three tier AD system with flexibility to optimize any specific running parameter of the AD plant and ensuring unimpaired optimal running of the microbial digestion processes
- A selected set of industrial unit operations enabling the functionality of the Method & Plant

The list is not exhaustive.

The present invention provides for an extraordinarily efficient method and plant for carrying out the method, for anaerobic digestion of lignocellulosic raw material. It has surprisingly been found that the method and plant provided by the present invention can provide high levels of anaerobic digestion of lignocellulosic raw material, even when the lignocellulose raw material is supplied as single feedstock or as a supplement to a co-digestion plant also digesting other organic wastes or slurries. The present invention provides a method and a plant for carrying out the method that, as a beneficial additional effect, is capable of highly effectively digest these other additional substrates.

The present invention provides a method and a plant for carrying out the method for anaerobic digestion of lignocellulosic raw material, wherein the lignocellulosic matter is digested to the effect of 80-90% of the theoretical methane potential of the lignocellulosic raw material utilized. In one embodiment of the present invention the remaining energy potential of the remaining organics is utilized as a heat source, by subjecting the remaining organics to a wet oxidation thus achieving a virtual 100% destruction and utilization of the organic matter.

The method and a plant for carrying out the method for anaerobic digestion of lignocellulosic raw material provided by the present invention thus provide result ensuring minimal losses of organic matter such as lignin degradation products, fertilizer end products or other organic byproducts of less value.

The present invention provides a method and a plant for carrying out the method for anaerobic digestion of lignocellulosic raw material, with high efficiency realized by very short retention times of the lignocellulosic raw material or other substrates utilized, high organic loading rates, and high specific gas production rates per weight unit of lignocellulosic raw material or other feedstock utilized. Furthermore, the plant provided by the present invention is highly effective, demonstrating exceptionally high specific gas production per reactor volume compared with prior art. This is not only applicable for the gas production but applies for any other plant parameter such as gas yield per invested capital. For example, the methane yield per tons of liquid animal manures is typically around 20 Nm3 methane per tons bulk weight, while the methane yield per bulk tons of cellulosic matter is around 400 Nm3 per tons. Any plant with a major fraction of cellulosic matter as feedstock and based on the present invention produces much more biogas per plant, as clearly demonstrated in the data above. This exceptional performance can be measured in gas output per invested capital or gas output per m3 reactor volume or any other comparison to prior art.

The present invention provides a method and a plant for carrying out the method for anaerobic digestion of lignocellulosic raw material in large quantities. One embodiment of the present invention provides a single feedstock anaerobic digestion plant with annual capacities in terms of feedstock ranging from 150.000 metric tons to 1 million tons, or above 1 million tons. As disclosed above, by combining the disclosure of the present invention, the process of densification of cellulosic matter by briquetting according to WO 2013/15771 (or with other pretreated lignocellulosic raw material demonstrating similar characteristic), the present invention provides a breakthrough in anaerobic digestion, further providing a method and a plant capable of bringing industrialization of anaerobic digestion of lignocellulosic raw material to new heights and possibilities. Further embodiments of the Method & Plant include, but are not limited to, conversion of biogas to methanol, ethanol, jet fuel, diesel, naphtha and other biofuels through a variety of chemical synthesis processes.

Yet another surprising and beneficial effect gained by the use of the present invention is that the present invention provides a method and a plant for carrying out the method for producing biogas, capable of virtual 100% of VS or virtually with a 100% energy efficiency.

This virtual 100% destruction can broadly be divided into e.g. 90% digestion of the biomass, resulting in methane and CO2 and e.g. 10% oxidation into heat and CO2.

The present invention provides a method and a plant for carrying out the method for producing biogas by anaerobic digestion of lignocellulosic raw material wherein over 90%, such as between 90-100%, such as above 95% of the Volatile Solids are utilized for production, that is a method and a plant that has above 90% energy efficiency.

The present invention provides a method and a plant for carrying out the method for producing biogas by anaerobic digestion of lignocellulosic raw material, wherein there is less than 10% energy loss, such as below 5%, such as between 0.1-5% energy loss.

The present invention provides a method and a plant for carrying out the method for producing biogas by anaerobic digestion of lignocellulosic raw material capable of producing biogas with over 90% efficiency, such as between 90-100%, such as above 95%, such as between 95-99% such as with 90-99% energy efficiency.

## Claims

1. A method for producing biogas from an organic raw material comprising at least 40% w/w lignocellulose biomass, the method comprising the steps of:
(a) providing an organic raw material (101) comprising at least 40% lignocellulose biomass;
(b) forming a biomass slurry (2, 110) comprising the organic raw material (101) and liquid comprising at least one of: fresh water (102), combined aqueous recirculation stream (144), recycled condensate stream (143), recycled first retentate stream (132) or recycled stream (121) comprising at least part of thick fraction and at least part of third digestate, and wherein process steps (b) and (c) can be two separate sequential process steps or one combined process step;
(c) subjecting the biomass slurry (2, 110) to a first fermentation process step, wherein the first fermentation is a hyperthermophillic anaerobic fermentation, thereby producing a first biogas stream (180) and a first digestate (111);
(d) subjecting the first digestate (111) to a second fermentation process step, wherein the second fermentation is a thermophilic anaerobic fermentation, thereby producing a second biogas stream (181) and a second digestate (112);
(e) subjecting the second digestate (112) to solid/liquid separation process, thereby producing a thin fraction (113) and a thick fraction (120);
(f) recycling at least part of the thick fraction (120) into the first fermentation step (c) and/or into the second fermentation step (d) and/or withdraw at least part of the thick fraction (120) from the process;
and either:
(g) optionally subjecting at least part of the thin fraction (113) to a third fermentation process step, wherein the third fermentation is an anaerobic digestion, thereby producing a third biogas stream (182) and a third digestate (114) and wherein optionally a part of the third digestate (114) is recycled into the first fermentation (c) and/or second fermentation (d) step, and/or bypass at least part of the thin fraction (113) directly to the first filtration process step
(h) thereby producing a first permeate (115) and a first retentate (130a);
(h) subjecting at least part of third digestate (114) to a first filtration process step, thereby producing a first permeate (115) and a first retentate (130a);
(i) optionally subjecting at least part of the first retentate (130a) to a wet oxidation process step, thereby producing an oxidated first retentate (131) and/or heat and/or inorganic byproduct, and/or withdraw at least part of the first retentate (130a) as a byproduct (191);
(j) when oxidated first retentate (131) is produced, recycling at least part of the oxidated first retentate (131), into the first fermentation step (c) and/or into the third fermentation (g);
(k) subjecting at least part of the first permeate (115) to a second filtration process step, thereby producing a second permeate (142) and a second retentate (116);
(l) recycling at least part of the second permeate (142) into the first fermentation (c); and
(o) collecting biogas (180, 181, 182) from first, second and third fermentation steps (c, d, g)
or:
(gg) bypassing process steps (g)-(n) by connecting the thin fraction (113) stream and optionally a part of the thick fraction (120) stream to another anaerobic digestion plant as additional lignocellulose biomass raw material; or by withdrawing the thin fraction (113) and at least part of the thick fraction (120) from the process; and
(oo) collecting biogas (180, 181);
wherein the first fermentation step (c) takes place at temperature between 60-85 degree Celsius and the second fermentation step (d) takes place at temperature between 45-65 degree Celsius.

2. A method according to claim 1, wherein the second retentate (116) is subjected to an evaporation process step (m), thereby producing a concentrated vinasse stream (190) and a condensate stream (143), and wherein at least part of the condensate stream (143) is recycled (n) into the first fermentation step (c).

3. A method according to either claim 1 or 2, wherein the heat produced by the wet oxidation step (i) is utilized for pre-heating the first retentate (130b) stream before subjecting it to the wet oxidation step (i); and/or for supplying heat into the first fermentation (c) and/or supplying heat for external heat consumption.

4. A method according to any of the preceding claims, wherein over 80% of the raw material comprising at least 40% w/w lignocellulose biomass is digested.

5. A method according to any of the preceding claims, wherein the biological raw material (101) comprises at least 80% lignocellulose biomass.

6. A method according to any of the preceding claims wherein the biological raw material (101) comprises between 90-100% lignocellulose biomass.

7. A method according to any preceding claims, wherein above 80% of all liquid used in process step (b) comprises one or more of combined aqueous recirculation stream (144), recycled condensate stream (143), recycled first retentate stream (132), or recycled stream (121) comprising at least part of thick fraction and at least part of third digestate.

8. A method according to claim 1, wherein in process step (gg) the stream of the thin fraction (113) is subjected to precipitation step and wherein the supernatant is recycled into the first fermentation step (c) together with at least part of the thick fraction (120).

9. A system for producing biogas from an organic raw material comprising at least 40% w/w lignocellulose biomass, the system comprising:
- a first reactor (3), configured for digesting a biomass slurry (2, 110) comprising biological raw material (101) comprising at least 40% w/w lignocellulose biomass mixed with liquid comprising at least one of: fresh water (102), combined aqueous recirculation stream (144), recycled condensate stream (143), recycled first retentate stream (132), or recycled stream (121) comprising at least part of thick fraction and at least part of third digestate, by hyperthermophillic anaerobic fermentation, thereby producing a first biogas (180) and a first digestate (111);
- a second reactor (4) configured for receiving and anaerobically digesting the first digestate (111) by thermophilic anaerobic fermentation, thereby producing a second biogas (181) and a second digestate (112);
- a solid/liquid separator (5), configured for receiving and processing the second digestate (112) and for producing a thick fraction (120) and a thin fraction (113);
and optionally:
- a third reactor (6) configured for receiving and anaerobically digest the thin fraction (113) by anaerobic digestion, thereby producing a third biogas (182) and a third digestate (114);
and:
- a first filtration unit (7) configured for receiving and filtering at least part of the third digestate (114), thereby producing a first permeate (115) and a first retentate (130a);
- a wet oxidation unit (9) configured for receiving and oxidating at least part of the first retentate (130a), thereby producing an oxidated first retentate (131);
- a second filtration unit (8), configured for receiving and filtering at least part of the first permeate (115), thereby producing a second permeate (142) and a second retentate (116);
- a biogas collecting system (10), configured for collecting biogas (180,) from the first reactor (3) and from the second reactor (4) (181) and optionally biogas (182) from the third reactor (6); and
a recycling system for recycling over 50% of total liquid in the system, comprising means constructed for:
- Recycling stream (132) comprising at least part of oxidated first retentate (131) and/or at least part of first retentate (130b) into the first reactor (3) and/or the third reactor (6);
- Recycling stream (121) comprising at least part of third digestate (114) and/or at least part of thick fraction (120) into the first reactor (3) and/or the second reactor (4);
- Recycling stream (144) comprising at least part of recycling stream (141) and/or at least part of second permeate (142) stream (143) comprising at least part of the condensate from the evaporation unit((14).

10. A system according to claim 9, wherein the organic raw material comprises at least 80% w/w lignocellulose biomass and wherein the recycling means are capable of recycling over 70% of total liquid in the system.

11. A system according to claim 9 or 10, wherein the organic raw material comprises over 90% w/w lignocellulose biomass and wherein the recycling means are capable of recycling over 80% of total liquid in the system.

12. A system according to claim 9-11, wherein the liquid in biomass slurry (2) within the first reactor (3) comprises above 80% of one or more of combined aqueous recirculation stream (144), recycled condensate stream (143), recycled first retentate stream (132), or recycled stream (121) comprising at least part of thick fraction and at least part of third digestate.

13. A system according to claim 9 for performing the method of claim 1.
